# EUROPEAN PATENT APPLICATION

(11) **EP 4 691 538 A1**
(43) Date of publication of application: **11.02.2026**
(21) Application number: 24784267.7
(22) Date of filing: 02.04.2024
(51) Int. Cl.: A61M 31/00, A61J 7/00

(54) **ORAL MEDICATION DISPENSER, MANUFACTURING METHOD THEREFOR AND USE THEREOF**

(30) Priority: 04.04.2023 CN 202310366350; 04.04.2023 CN 202320730369 U
(71) Applicant: Shanghai WD Pharmaceutical Co., Ltd, Shanghai 201203 (CN)
(72) Inventor: FAN, Ming, Shanghai 201203 (CN); DONG, Liangchang, Shanghai 201203 (CN); LIU, Zhepeng, Shanghai 201203 (CN); WEI, Xiaoxiong, Shanghai 201203 (CN)
(74) Representative: Greaves Brewster LLP
(86) International application number: PCT/CN2024/085477
(87) International publication number: WO 2024/208183

(57) **Abstract**

Disclosed in the present invention are an oral medication dispenser, a manufacturing method therefor and a use thereof. The oral medication dispenser comprises a core assembly and a fixing device; the core assembly comprises a molar matching functional area and a medication carrying functional area which are integrally formed; the molar matching functional area comprises a first section close to the medication carrying functional area and a second section far away from the medication carrying functional area; one end of the first section is connected to one end of the second section, and the other end of the first section and the other end of the second section are open ends, so as to form a U-shaped structure; the first section and the second section match the buccal side and the lingual side of teeth, respectively; the medication carrying functional area comprises a first ring and a second ring which are coaxially arranged; the fixing device is used for enhancing the fixing effect of the core assembly on the teeth; and the fixing device is a fixator or a fixing layer. When in use, the oral medication dispenser has high wearing firmness, high safety, high comfort and a small influence on the appearance of the face.

## Description

The present application claims the priorities of Chinese patent application CN2023103663509 filed on April 4, 2023 and Chinese patent application CN2023207303692 filed on April 4, 2023. The contents of the above patent applications are incorporated herein by reference in their entireties.

### TECHNICAL FIELD

The present disclosure relates to an oral cavity medication delivery device, a manufacturing method therefor and a use thereof.

### BACKGROUND

For active pharmaceutical ingredients such as levodopa, carbidopa, baclofen, and acyclovir, whose absorption windows are limited at the upper gastrointestinal tract, conventional sustained-release preparations have compromised bioavailability and insufficient therapeutic coverage. Existing technologies for extending the retention time in the stomach of drugs (including expansion, swelling, floating, raft-forming, sinking and muco-adhesion) have very limited prolonging effects, particularly when administered at the fasting state. In addition, for topical administration to the oral mucosa, liquid or semisolid preparations such as conventional sprays, gargles, and ointments have short retention time in the oral cavity and cannot achieve a sustained drug administration effect. Therefore, there is a need for a drug delivery system that could provide long-term exposure of pharmaceutical ingredients, and is suitable for drugs with absorption windows in the upper gastrointestinal tract or requiring local administration in the oral cavity. The drug delivery device is loaded with drugs and fixed in the oral cavity after being combined with a fixator to form a drug controlled-release system that provides long-term exposure of drugs with their absorption window limited to the upper gastrointestinal tract.

Regarding oral cavity medication delivery devices, US10668274 and CN1997421A disclose an oral drug containing container based on an electrically controlled drug release mechanism. The drug-containing container and the electric control system they claimed are difficult to implement. CN105873631A discloses an oral cavity medication delivery device that requires an electronic or mechanical pump as external power for drug delivery. In addition, CN1925823A discloses a dental bracket for attaching fluoride pills to teeth to improve the treatment or prevention of dental caries, and this patent application does not involve drug absorption in the upper gastrointestinal tract. Furthermore, CN109908461A discloses an oral cavity medication delivery device applied to a false tooth, tooth braces or an oral implant, which is suitable for administration of oral mucosa and does not involve drug absorption in the upper gastrointestinal tract. The above disclosed patent applications may require external power, or rely on a false tooth, tooth braces, etc., for realization, or do not involve drug absorption at the upper gastrointestinal tract.

CN114191307A and CN212973560U disclose an oral retention device for retaining the medicinal tablets in the oral cavity, providing long-term exposure of drugs with absorption windows limited to the upper gastrointestinal tract to obtain long-term stable blood drug concentrations. The oral retention device has taken into account that the medicinal tablets are inserted from back to front, close to the throat and are blocked by the oral tissues such as the buccal fat pad tip, so that the medicinal tablets are not easy to fall off when wearing the device. At the same time, the tooth-matching component in the oral retention device can fit with the patient's teeth according to personalized design, making it stable to wear. However, for patients with too short clinical crowns, small buccal space, or special oral conditions, when the mouth movement is large, it may cause poor retention of the oral retention device, and there is a risk of lifting or falling off.

The above oral cavity medication delivery devices do not take into account the firmness of wearing under special oral conditions, and that complex structures or large volume of device may result in poor wearing comfort and safety for patients, and may also have a significant impact on facial appearance when wearing.

### CONTENT OF THE PRESENT INVENTION

In order to solve the problem in the prior art that a drug delivery device is at a risk of falling off for some patients wearing a drug delivery device after substantially opening of mouth and other movements, the present disclosure provides an oral cavity medication delivery device, a manufacturing method therefor and a use thereof. Specifically, the present invention provides an oral cavity medication delivery device for medicinal tablet insertion from back to front, which is comprised of a core assembly and a fixing device, stably holds the medicinal tablet in the oral cavity, preventing it from falling out due to mouth movement. This oral cavity medication delivery device can achieve the long-term release of drugs whose absorption window is limited to the upper gastrointestinal tract, as well as oral mucosal delivery or localized oral drug release. Furthermore, the oral cavity medication delivery device provides high comfort and minimal impact on facial appearance during use.

The present disclosure solves the above-mentioned technical problems through the following technical solutions:
**The present disclosure provides an oral cavity medication delivery device,** which comprises a core assembly and a fixing device, and the core assembly comprises a molar matching functional area and a medication carrying functional area which are integrally formed;
When the oral cavity medication delivery device is fixed in the oral cavity, the medication carrying functional area is located in the space between the teeth and the cheek, or the medication carrying functional area is located in the space between the teeth and the tongue;
The molar matching functional area comprises a first section close to the medication carrying functional area and a second section far away from the medication carrying functional area; one end of the first section and the second section are connected to each other, and the other end is an open end, which forms a U-shaped structure; the first section and the second section are used to anastomose with the buccal and lingual sides of the teeth respectively, and are used to fix the medication carrying functional area on the teeth;
The medication carrying functional area comprises a first ring and a second ring arranged coaxially; the axial clamping space formed between the first ring and the second ring is used to fix the drug;
The fixing device is a fixator or a fixing layer, used to strengthen the fixing effect of the core assembly on the teeth;
When the fixing device is a fixator:
   When the oral cavity medication delivery device is in use, the lingual, buccal and occlusal surfaces of the fixator correspond to the lingual, buccal and occlusal surfaces of the teeth and cover the outer peripheral surface of the teeth; the fixator simultaneously covers the molar matching functional area;
   When the fixing device is a fixing layer:
      The fixing device is attached to the outer surfaces of the first section and the second section for contact with teeth.

In the present disclosure, the first ring and the second ring are preferably located on the buccal side of the first or second molar.

In the present disclosure, the fixator and the molar matching functional area are preferably connected by means of mechanical assembly, mechanical connection or adhesive bonding.

Herein, the mechanical assembly is preferably embedment, and slotted holes are provided in the fixator at the position corresponding to the medication carrying functional area on the fixator to achieve removable fixation between the fixator and the core assembly. Embedment means that the molar matching functional area is embedded in the space formed by both sides of the fixator.

Herein, the mechanical connection is preferably welding, riveting or bolting.

Herein, the adhesive is preferably one or more selected from pressure-sensitive adhesive, starch glue, latex, epoxy resin and polyurethane acrylate.

In the present disclosure, the fixing layer is preferably also attached to other outer surfaces of the first section and the second section.

In the present disclosure, the first ring is close to the open end of the molar matching functional area, and the second ring is far away from the open end of the molar matching functional area. The first ring and the second ring are preferably solid or hollow rings.

Herein, the hollow ring is a closed ring or an open ring.

Herein, the shape of the ring is preferably one or more selected from circle, ellipse, and polygon.

Herein, the shapes of the ring of the first ring and the second ring are preferably circular; the hollow area of the first ring is preferably smaller than that of the second ring, and the medicinal tablet can be inserted from the second ring to the first ring.

In the present disclosure, the material of the core assembly is preferably one of titanium, stainless steel, cobalt chromium alloy, cobalt chromium molybdenum alloy or precious metal, more preferably cobalt chromium alloy, and the materials of the core assembly are all meet the standards for dental materials.

In the present disclosure, the material of the fixator is preferably one or more selected from polyvinyl chloride, polyethylene terephthalate, polyethylene terephthalate-1,4-cyclohexanedimethpolyvinyl chloride, polyurethane, polyamide, ethylene-vinyl acetate copolymer, polycaprolactone, high-density polyethylene, polypropylene, epoxy acrylate, methacrylate, and polyurethane acrylate; more preferably, polyethylene terephthalate or ethylene-vinyl acetate copolymer, the materials of the fixator all meet standards for medical grade.

In the present disclosure, the material of the fixing layer is preferably one or more selected from polyvinyl chloride, polyethylene terephthalate, polyethylene terephthalate-1,4-cyclohexanedimethylene terephthalate, polyurethane, polyamide, ethylene-vinyl acetate copolymer, polycaprolactone, high-density polyethylene, polypropylene, cellulose acetate, hydroxypropyl cellulose and copovidone; more preferably, polyethylene terephthalate or copovidone, the materials of the fixing layer all meet standards for medical grade.

In the present disclosure, the thickness of the fixing layer is preferably 0.01mm-1mm.

In the present disclosure, the number of teeth covered by the fixator can be adjusted according to the condition of the teeth and the wearing firmness. The length of the fixator is preferably corresponding to the length of 4-16 teeth in the upper jaw or lower jaw, and more preferably is corresponding to the length of 5-9 teeth in the maxillary and mandible.

In the present disclosure, the length of the molar matching functional area is preferably corresponding to the length of 2-5 teeth in the mandible.

Those skilled in the art would understand that the length is a length that enables the fixator or the molar matching functional area to cover the number of teeth, and does not exceed the number of teeth within the target range.

In a preferred embodiment, the fixator is connected to the molar matching functional area in an embedded manner, and slotted holes are provided in the retainer at the position corresponding to the medication carrying functional area on the fixator to achieve removable fixation between the fixator and the core assembly; the length of the molar matching functional area is corresponding to the length of the mandibular first and second premolars and the first and second molars; the first ring and the second ring are circular closed rings.

In a preferred embodiment, the fixator is connected to the molar matching functional area in an embedded manner; the length of the molar matching functional area is corresponding to the length of the mandibular first and second premolars and the first and second molars; the first ring and the second ring are elliptical closed rings.

In a preferred embodiment, the fixator is connected to the molar matching functional area in an embedded manner; the length of the molar matching functional area is corresponding to the length of the maxillary second premolar and the first, second, and third molars; the first ring and the second ring are circular closed rings.

In a preferred embodiment, the fixator is connected to the molar matching functional area using pressure-sensitive adhesive as an adhesive; the length of the molar matching functional area is corresponding to the length of the first, second and third molars; the first ring and the second ring are polygonal closed rings.

In a preferred embodiment, the fixing layer is attached to all outer surfaces of the first section of the U-shaped structure and the second section of the U-shaped structure; the length of the molar matching functional area is corresponding to the length of the mandibular first and second premolars and the first and second molars; the first ring and the second ring are circular closed rings.

In a preferred embodiment, a pair of clamping walls are symmetrically provided on the first ring in the direction of the open end of the molar matching functional area.

**The present disclosure further provides a manufacturing method for the above-mentioned oral cavity medication delivery device,** which comprises the following steps: when the fixing device is a fixator, combining the fixator with the integrally formed first section and the second section in a wrapped form; when the fixing device is a fixing layer, the fixing layer is attached to the outer surfaces of the integrally formed first section and second section.

In the present disclosure, a method for preparing the core assembly preferably comprises laser casting, injection molding or impression molding.

Herein, the steps of laser casting are preferably as follows: scanning the subject's oral cavity or taking an oral mold to make a plaster model and then scanning to obtain the subject's oral data; using three-dimensional design software and dental design software to respectively design the medication carrying functional area and the molar matching functional area of the core assembly, and combining them in the dental design software to generate a design file for the core assembly; and preparing the core assembly by laser casting, grinding, polishing, and cleaning.

Herein, the steps of injection molding are preferably as follows: taking an oral mold of the subject to make a plaster model; preparing a dental wax model on the plaster model using dental wax as a material, and preparing the core assembly through a traditional injection molding process.

In the present disclosure, a method for preparing the fixator preferably comprises thermoforming, impression, cutting or 3D printing.

Herein, the steps of thermoforming are preferably as follows: heating and softening the raw material of the fixator through a thermoforming machine and then vacuuming, and covering the raw material of the fixator on the dental mold and the molar matching functional area.

Herein, the steps of impression are preferably as follows: heating and softening the raw material of the fixator, and covering it on the subject's teeth and the molar matching functional area.

In the present disclosure, the raw material of the fixator is preferably as described above.

In the present disclosure, a method for preparing the fixing layer preferably comprises dipping, spraying or thermoforming.

Herein, the steps of dipping are preferably as follows: after dissolving the raw material of the fixing layer, immersing the molar matching functional area into the raw material solution of the fixing layer, taking it out, and drying it to form a membrane.

Herein, the steps of spraying are preferably as follows: after dissolving the raw material of the fixing layer, coating it to the molar matching functional area by spraying, and drying it to form a membrane.

Herein, the steps of thermoforming are preferably as follows: heating and softening the raw material of the fixing layer, covering it on the anastomosis functional area, and then cooling it.

In the present disclosure, the raw material of the fixing layer is preferably as described above.

In the present disclosure, the wrap structure of the fixator and the first section and the second section is preferably formed by mechanical assembly, mechanical connection or adhesive bonding.

Herein, the mechanical assembly is preferably in the form of embedment.

When the combination method is mechanical assembly, the fixator fits with the dental mold and the core assembly during the molding process, naturally forming a slot-like structure. At the same time, a buckle design can be added to the molar matching functional area as needed.

Herein, the mechanical connection is preferably in the form of welding, riveting or bolting.

Herein, the coating position of the adhesive is preferably the outer surfaces of the first section and the second section for contact with the teeth, and more preferably is the entire outer surfaces of the first section and the second section.

Herein, the coating thickness of the adhesive is preferably no more than 1.0 mm.

**The present disclosure further provides a use of the above-mentioned oral cavity medication delivery device,** characterized in that: when the fixing device is a fixator, anastomosing the lingual, buccal and occlusal sides of the fixator with the lingual, buccal and occlusal surfaces of the teeth and wrapping around the peripheral surfaces of the teeth; placing the medication carrying functional area in the space between the teeth and the cheek, or placing the medication carrying functional area in the space between the teeth and the tongue; and wrapping the molar matching functional area with the fixator;
When the fixing device is a fixing layer: placing the medication carrying functional area in the space between the teeth and the cheek, or placing the medication carrying functional area in the space between the teeth and the tongue.

Without violating the common knowledge in the art, the preferred conditions described above may be optionally combined to obtain the preferred embodiments in the present disclosure.

The reagents and raw materials employed in the present disclosure are commercially available.

The positive effects of the present disclosure lie in:
The present disclosure provides an oral cavity medication delivery device which matches with the teeth in the oral cavity and has an extremely low risk of falling off. Inserting the medicinal tablet into the medication carrying functional area of the drug delivery device from the back to the front, and fixing the oral cavity medication delivery device to the corresponding teeth in the oral cavity after combining the medicinal tablet to achieve the sustained release of the drug. The drug comprises, but is not limited to, drugs whose absorption window is limited to the upper gastrointestinal tract, and drugs that act locally in the oral cavity;
The oral cavity medication delivery device of the present disclosure does not require external power, nor does it require adjustments of the patient's teeth, and is easy to wear and operate. The oral cavity medication delivery device of the present disclosure has high wearing firmness, i.e., high safety, which can avoid accidental swallowing or choking caused by loosening and dislodging of the drug delivery device; the oral cavity medication delivery device of the present disclosure has little impact on the facial appearance of the patient when wearing, and has high wearing aesthetics; and at the same time, the oral cavity medication delivery device also has high comfort.

### BRIEF DESCRIPTION OF THE DRAWINGS

Figure 1 is a schematic diagram of each component of the oral cavity medication delivery device of Embodiment 1 of the present disclosure, which is composed of a core assembly 1 and a fixing device 2, wherein the core assembly 1 comprises a molar matching functional area 11 and a medication carrying functional area 12, and the medication carrying functional area 12 is composed of a first ring 121 and a second ring 122.
Figure 2 is a schematic diagram of the core assembly in the oral cavity medication delivery device of Embodiment 1 of the present disclosure.
Figure 3 is a schematic diagram of a fixator in the oral cavity medication delivery device of Embodiment 1 of the present disclosure when the fixing device is a fixator.
Figure 4 is a cross-sectional view taken along plane A-A in Fig. 1.
Figure 5 is a schematic diagram of the oral cavity medication delivery device of Embodiment 2.
Figure 6 is a schematic diagram of the oral cavity medication delivery device of Embodiment 3.
Figure 7 is a schematic diagram of the oral cavity medication delivery d device of Embodiment 6.
Figure 8 is a cross-sectional view taken along plane B-B of Fig. 7.
Figure 9 is a schematic diagram of the oral cavity medication delivery device of Embodiment 9.

List of reference numerals:
1-Core assembly; 2-Fixing device; 201-Lingual side of the fixing device; 202-Buccal side of the fixing device; 203-Occlusal surface of the fixing device; 11-Molar-fittingfunctional area; 12-Medication carrying functional area; 121- First ring; 122-Second ring; 123-Clamping wall.

### DETAILED DESCRIPTION OF THE PREFERRED EMBODIMENT

The following examples further illustrate the present disclosure, but the present disclosure is not limited thereto. The experimental methods without specific conditions specified in the following embodiments were selected according to conventional methods and conditions, or according to product specifications.

### Embodiment 1

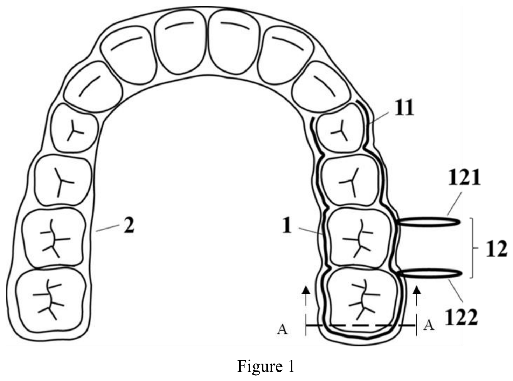

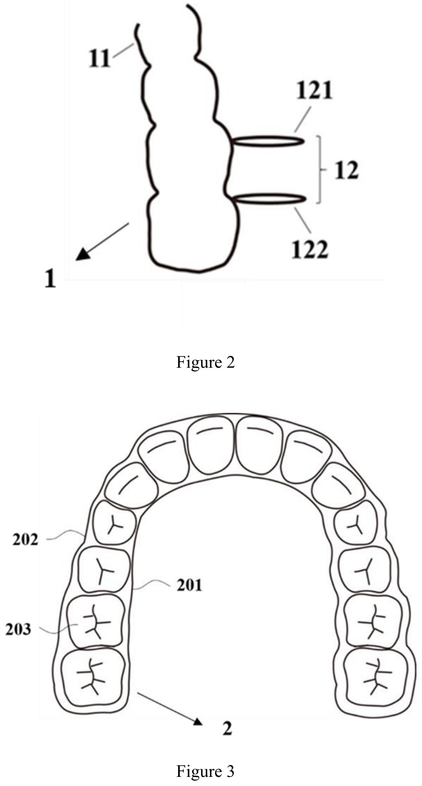

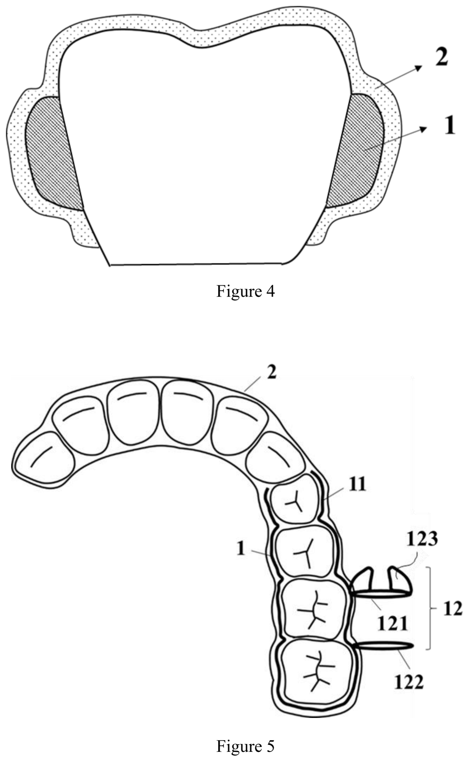

This embodiment is an oral cavity medication delivery device that covers all mandibular teeth. As shown in Figure 1, it comprises a core assembly 1 and a fixing device 2. The fixing device 2 is a fixator. The structural schematic diagram of the core assembly 1 is shown in Figure 2, the structural schematic diagram of the fixing device 2 is shown in Figure 3, and the cross-sectional view of the oral cavity medication delivery device is shown in Figure 4. The lingual side 201 of the fixing device, the buccal side 202 of the fixing device and the occlusal surface 203 of the fixing device correspondingly anastomose to the lingual side, buccal side and occlusal surface of the teeth and cover the outer peripheral surface of the teeth; the fixing device 2 covers molar matching functional area 11 at the same time. The core assembly 1 is composed of a molar matching functional area 11 and a medication carrying functional area 12. The molar matching functional area 11 can be closely matched with the mandibular first and second premolars and the first and second molars in the subject's oral cavity, and the size of the medication carrying functional area 12 allows it to accommodate at least one tablet and fix the tablet in the oral cavity. The fixing device 2 is tightly matched with all the mandibular teeth of the subject and with the molar matching functional area 11, so that the core assembly 1 and the tablet are fixed in the oral cavity. Both the molar matching functional area 11 and the medication carrying functional area 12 are made of cobalt-chromium alloy, and the molar matching functional area 11 and the medication carrying functional area 12 are connected on respective sides. The medication carrying functional area 12 comprises a first ring 121 and a second ring 122. The first ring 121 and the second ring 122 are circular closed rings. The tablet is inserted from the second ring 122 and blocked by the first ring 121. The molar matching functional area 11 and the fixing device 2 are combined by embedding through mechanical assembly method. The fixing device 2 is provided with slotted holes at a location corresponding to the medication carrying functional area 12 to achieve removable fixation between the fixing device 2 and the core assembly 1. The fixing device 2 is made of colorless and transparent polyethylene terephthalate and covers all the mandibular teeth, that is, covers 14 teeth. The core assembly 1 and the fixing device 2 are combined to match the teeth in the subject's oral cavity.

The oral cavity medication delivery device of this embodiment can be worn firmly in the oral cavity after holding the tablets, the tablets are fixed in the oral cavity, and the fixator, the core assembly and the tablets will not fall off or shift with the actions of the oral cavity. The oral cavity medication delivery device has high wearing comfort, and has little impact on facial appearance.

### Embodiment 2

This embodiment is an oral cavity medication delivery device covering 10 mandibular teeth. As shown in Figure 5, it comprises a core assembly 1 and a fixing device 2, the fixing device 2 is a fixator. The core assembly 1 is composed of a molar matching functional area 11 and a medication carrying functional area 12. The molar matching functional area 11 can be closely matched with the mandibular first and second premolars and the first and second molars in the subject's oral cavity, and the size of the medication carrying functional area 12 allows it to accommodate at least one tablet and fix the tablet in the oral cavity. The fixing device 2 is tightly matched with part of the subject's mandibular teeth (including the teeth from the left canine to the right second molar), and is tightly fitted with the molar matching functional area 11, so that the core assembly 1 and the tablet are fixed in the oral cavity. Both the molar matching functional area 11 and the medication carrying functional area 12 are made of cobalt-chromium alloy, and the molar matching functional area 11 and the medication carrying functional area 12 are connected on respective sides. The medication carrying functional area 12 comprises a first ring 121 and a second ring 122. The first ring 121 and the second ring 122 are elliptical closed rings. A pair of clamping walls 123 are symmetrically provided on the first ring 121 in the direction of the open end of the molar matching functional area, which is suitable for inserting non-cylindrical shaped tablets. The tablets are inserted from the second ring 122 and blocked by the first ring 121. The molar matching functional area 11 and the fixing device 2 are combined by embedding through a mechanical assembly method. The fixing device 2 is provided with slotted holes at a location corresponding to the medication carrying functional area 12 to achieve removable fixation between the fixing device 2 and the core assembly 1. The fixing device 2 is made of colorless and transparent polyethylene terephthalate and covers 10 teeth of the mandible from the left canine to the right second molar. The core assembly 1 and the fixing device 2 are combined to match with part of the mandibular teeth in the subject's oral cavity.

The oral cavity medication delivery device of this embodiment can be worn firmly in the oral cavity after holding the tablets, the tablets are fixed in the oral cavity, and the fixator, the core assembly and the tablets will not fall off or shift with the actions of the oral cavity. The oral cavity medication delivery device has high wearing comfort, and has little impact on facial appearance.

### Embodiment 3

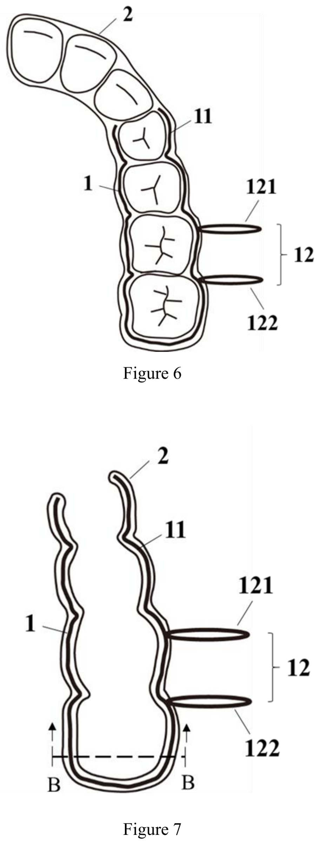

This embodiment is an oral cavity medication delivery device covering 7 mandibular teeth. As shown in Figure 6, it comprises a core assembly 1 and a fixing device 2, the fixing device 2 is a fixator. The core assembly 1 is composed of a molar matching functional area 11 and a medication carrying functional area 12. The molar matching functional area 11 can be closely matched with the mandibular first and second premolars and the first and second molars in the subject's oral cavity, and the size of the medication carrying functional area 12 allows it to accommodate at least one tablet and fix the tablet in the oral cavity. The fixing device 2 is tightly matched with part of the mandibular teeth (including a total of 7 teeth from the right incisor to the right second molar), and is tightly fitted with the molar matching functional area 11, so that the core assembly 1 and the tablet are fixed in the oral cavity. Both the molar matching functional area 11 and the medication carrying functional area 12 are made of cobalt-chromium alloy, and the molar matching functional area 11 and the medication carrying functional area 12 are connected on respective sides. The medication carrying functional area 12 comprises a first ring 121 and a second ring 122. The first ring 121 and the second ring 122 are circular closed rings and are suitable for inserting cylindrical-shaped tablets. The tablets are inserted from the second ring 122 and blocked by the first ring 121. The molar matching functional area 11 and the fixing device 2 are combined by embedding through a mechanical assembly method. The fixing device 2 is provided with slotted holes at a location corresponding to the medication carrying functional area 12 to achieve removable fixation between the fixing device 2 and the core assembly 1. The fixing device 2 is made of colorless and transparent ethylene-vinyl acetate copolymer and covers a total of 7 teeth on the right side of the mandible from the incisor to the second molar. The core assembly 1 and the fixing device 2 are combined to match the subject's teeth in the subject's oral cavity.

Wearing the oral cavity medication delivery device of this embodiment in the oral cavity, after being loaded with the tablets, the tablets can be fixed in the oral cavity and not easy to fall off. The wearing firmness is not significantly different from that of Embodiments 1 and 2. And at the same time, because the number of teeth covered is small, the wearing comfort and the degree of influence on facial appearance is better than that of Embodiments 1 and 2.

### Embodiment 4

The difference between this embodiment and Embodiment 1 is that the oral cavity medication delivery device covers all of the maxillary teeth, and the molar matching functional area 11 can be closely matched with the maxillary second premolar and the first, second, and third molars in the subject's oral cavity. Others are the same as Embodiment 1.

The oral cavity medication delivery device of this embodiment can be worn firmly in the oral cavity after holding the tablets, the tablets are fixed in the oral cavity, and the fixator, the core assembly and the tablets will not fall off or shift with the actions of the oral cavity. The oral cavity medication delivery device has high wearing comfort, and has little impact on facial appearance.

### Embodiment 5

The difference between this embodiment and Embodiment 1 is that the length of the core assembly 1 corresponds to 3 teeth and can be designed for subjects with molar defects; the first ring 121 and the second ring 122 are polygonal closed rings, and the molar matching functional area 11 and the fixing device 2 are combined by bonding with a pressure-sensitive adhesive as the adhesive, and the bonding position is the entire outer surface of the molar matching functional area 11, and the thickness of the adhesive is 0.1 mm; the fixing device 2 is prepared from colorless and transparent ethylene-vinyl acetate copolymer. Others are the same as Embodiment 1.

The oral cavity medication delivery device of this embodiment can be worn firmly in the oral cavity after holding the tablets, the tablets are fixed in the oral cavity, and the fixator, the core assembly and the tablets will not fall off or shift with the actions of the oral cavity. The oral cavity medication delivery device has high wearing comfort, and has little impact on facial appearance.

### Embodiment 6

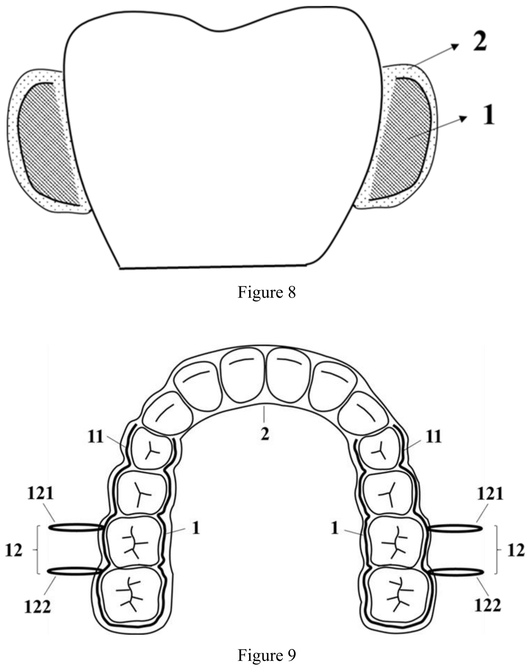

The structural schematic diagram of the oral cavity medication delivery device of this embodiment is shown in Figure 7. It comprises a core assembly 1 and a fixing device 2. The fixing device 2 is a fixing layer. The cross-sectional view of the oral cavity medication delivery device is shown in Figure 8. The core assembly 1 is composed of a molar matching functional area 11 and a medication carrying functional area 12. The molar matching functional area 11 can be closely matched with the mandibular first and second premolars and the first and second molars in the subject's oral cavity. The drug holding functional area comprises a first ring 121 and a second ring 122. The first ring 121 and the second ring 122 are circular closed rings and are suitable for inserting cylindrical-shaped tablets. The tablets are inserted from the second ring 122 and blocked by the first ring 121. The size of medication carrying functional area 12 allows it to accommodate at least one tablet and fix the tablet in the oral cavity. The fixing device 2 is attached to all outer surfaces of the first section of the U-shaped structure and the second section of the U-shaped structure; the molar matching functional area 11 and the medication carrying functional area 12 are made of cobalt-chromium alloy, and the molar matching functional area 11 and the medication carrying functional area 12 are connected at their respective sides. The fixing device 2 is made of colorless and transparent polyethylene terephthalate, which is softened by heating and then covered on the surface of the molar matching functional area 11, and is obtained by removing excess material after cooling. The thickness of the fixing device 2 is 0.3-0.5 mm, and the core assembly 1 and the fixing device 2 are combined to match the subject's teeth.

The oral cavity medication delivery device of this embodiment can be worn firmly in the oral cavity after being loaded with the tablets, and the tablets are fixed in the oral cavity, and the fixing layer, the core assembly and the tablets will not fall off or shift with the actions of the oral cavity. The oral cavity medication delivery device has high wearing comfort, and has little impact on facial appearance.

### Embodiment 7

The difference between this embodiment and Embodiment 6 is that the fixing device 2 is made of light blue polycaprolactone, and the thickness of the fixing device 2 is 0.8-1 mm. Others are the same as Embodiment 6.

The oral cavity medication delivery device of this embodiment can be worn firmly in the oral cavity after being loaded with tablets, and the tablets can be fixed in the oral cavity. However, as the thickness of the fixing device is relatively thick, the degree of wearing firmness and comfort of the oral cavity medication delivery device in this embodiment is not as good as that of Embodiment 6. Moreover, compared with Embodiment 6, this embodiment has a greater impact on facial appearance.

### Embodiment 8

The difference between this embodiment and Embodiment 6 is that the core assembly 1 and the fixing device 2 are bonded using pressure-sensitive adhesive. The bonding position is the part where the molar matching functional area is in contact with the teeth. The thickness of the adhesive is 0.1 mm; the fixing device 2 is a colorless and transparent polypropylene membrane with a thickness of 0.02-0.04 mm. Others are the same as Embodiment 6.

The oral cavity medication delivery device in this embodiment can be worn firmly in the oral cavity after being loaded with tablets, and the tablets can be fixed in the oral cavity. However, as the thickness of the fixing device is relatively thin and the surface of the fixing layer material is smooth, the degree of wearing firmness and comfort of the oral cavity medication delivery device in this embodiment is not as good as that of Embodiments 6 and 7.

### Embodiment 9

The oral cavity medication delivery device of this embodiment is shown in Figure 9, which comprises core assemblies 1 which are symmetrical on both sides and a fixing device 2, the fixing device 2 is a fixator. Two core assemblies 1 are composed of a molar matching functional area 11 and a medication carrying functional area 12. The molar matching functional area 11 can be closely matched with the maxillary first and second premolars and the first and second molars in the subject's oral cavity, and the size of the medication carrying functional area 12 allows it to accommodate at least one tablet and fix the tablet in the oral cavity. The fixing device 2 is tightly matched with all the maxillary teeth of the subject and with the molar matching functional area 11, so that the core assemblies 1 and the tablet are fixed in the oral cavity. Both the molar matching functional area 11 and the medication carrying functional area 12 are made of cobalt-chromium alloy, and the molar matching functional area 11 and the medication carrying functional area 12 are connected on respective sides. The medication carrying functional area 12 comprises a first ring 121 and a second ring 122. The first ring 121 and the second ring 122 are circular closed rings and are suitable for inserting cylindrical-shaped tablets. The tablets are inserted from the second ring 122 and blocked by the first ring 121. The molar matching functional area 11 and the fixing device 2 are combined by embedding through mechanical assembly method. The fixing device 2 is provided with slotted holes at a location corresponding to the medication carrying functional area 12 to achieve removable fixation between the fixing device 2 and the core assembly 1. The fixing device 2 is made of colorless and transparent polyethylene terephthalate and covers all the maxillary teeth. The core assemblies 1 and the fixing device 2 are combined to match the teeth in the subject's oral cavity.

The oral cavity medication delivery device of this embodiment can be worn firmly in the oral cavity after holding the tablets, the tablets are fixed in the oral cavity, and the fixator, the core assembly and the tablets will not fall off or shift with the actions of the oral cavity. The oral cavity medication delivery device has high wearing comfort, and has little impact on facial appearance.

### Embodiment 10

The laser casting method is used to prepare a cobalt-chromium alloy core assembly and the thermoforming method is used to prepare a fixator. The specific steps are as follows:
Step 1: obtaining the oral data of the subject by scanning the subject's oral cavity, or taking an oral mold to make a plaster model and then scanning;
Step 2: using 3D design software and dental design software to design the drug holding functional area and molar matching functional area of the core assembly respectively, and combining them in the dental design software to generate the core assembly design file;
Step 3: preparing the cobalt-chromium alloy core assembly through laser casting, grinding, polishing, and cleaning;
Step 4: wearing the core assembly on the subject's dental mold, heating and soften polyethylene terephthalate through a thermoforming machine and then vacuuming the dental mold, removing excess material and polishing, and removing it from the dental mold to obtain an oral cavity medication delivery device. The oral cavity medication delivery device matches the subject's teeth.

### Embodiment 11

The injection molding is used to prepare a cobalt-chromium alloy core assembly and the thermoforming method is used to prepare a drug delivery device. The specific steps are as follows:
Step 1: taking an oral mold of the subject and making a plaster model;
Step 2: preparing a dental wax model on a plaster model by using dental wax as the material;
Step 3: preparing a core assembly through traditional injection molding process by using cobalt-chromium porcelain alloy as material;
Step 4: wearing the core assembly on the plaster dental mold, heating and soften polyurethane through a thermoforming machine and then vacuuming the dental mold, removing excess material and polishing, and removing it from the dental mold to obtain the oral cavity medication delivery device with the combination of the core assemblies and the fixator.

### Embodiment 12

The injection molding is used to prepare a cobalt-chromium alloy core assembly and the impression method is used to prepare a drug delivery device. The specific steps are as follows:
Step 1: taking an oral mold of the subject and making a plaster model;
Step 2: preparing a dental wax model on a plaster model by using dental wax as the material;
Step 3: preparing the drug delivery device through traditional injection molding process by using cobalt-chromium porcelain alloy as material;
Step 4: wearing the core assembly on the subject's corresponding teeth, heating and soften polycaprolactone material, covering it on the subject's teeth and the molar matching functional area of the core assembly, and removing it from the teeth together with the core assembly after cooling to obtain the oral cavity medication delivery device.

### Comparative Embodiment 1

The oral cavity medication delivery device of this comparative embodiment only comprises a core assembly 1 in Embodiment 1, as shown in Figure 2.

### Test Embodiment 1

The oral cavity medication delivery devices of Embodiment 1, Embodiment 3, Embodiment 6 and Comparative Embodiment 1 are tested for wearing firmness, and the wearing firmness scores are shown in the following Table 1: wherein, 1 score is not firm, and means that it is very easy to fall off during basic oral actions such as opening the mouth and speaking after wearing; 2 score is less firm, and it sometimes loosens, buckles, or falls off under basic oral actions such as opening the mouth and speaking after wearing; 3 score is basically firm, and it is easy to fall off only during large oral actions; 4 score is firm, and it buckles or loosens only during large oral actions, but not fall off; 5 score is very firm, and it will not loosen, buckle or fall off during basic oral actions or large oral actions.

**Table 1**

| Subject | Embodiment 1 | Embodiment 3 | Embodiment 6 | Comparative Embodiment 1 |
|---|---|---|---|---|
| 1 | 5 | 5 | 4 | 3 |
| 2 | 5 | 5 | 5 | 5 |
| 3 | 5 | 5 | 5 | 4 |
| 4 | 5 | 5 | 5 | 3 |
| 5 | 5 | 5 | 4 | 2 |

As can be seen from Table 1, compared with Comparative Embodiment 1 which only uses the core assembly, Embodiment 1, Embodiment 3, and Embodiment 6 of the present disclosure which use a combination of the fixing device and the core assembly, have better wearing firmness, and the use of a fixator as the fixing device provides higher wearing firmness than the use of a fixing layer as the fixing device.

### Test Embodiment 2

The oral cavity medication delivery devices of Embodiment 1, Embodiment 3, Embodiment 6, and Comparative Embodiment 1 are tested for wearing comfort, and the wearing comfort scores are shown in the following Table 2: wherein, 1 score is extremely uncomfortable, there is obvious foreign matter sensation or irritation that is unacceptable; 2 score is uncomfortable, there is relatively obvious foreign matter sensation or irritation that is less acceptable; 3 score, three is foreign matter sensation or irritation that has a slight impact on the oral function, but is acceptable; 4-score is relatively comfortable, there is no obvious foreign matter sensation or irritation that has essentially no impact on oral function; and 5 score is very comfortable, there is almost no foreign matter sensation or irritation that has no impact on the oral function.

**Table 2**

| Subject | Embodiment 1 | Embodiment 3 | Embodiment 6 | Comparative Embodiment 1 |
|---|---|---|---|---|
| 1 | 3 | 4 | 4 | 3 |
| 2 | 4 | 5 | 5 | 5 |
| 3 | 3 | 4 | 4 | 3 |
| 4 | 4 | 5 | 4 | 3 |
| 5 | 3 | 4 | 4 | 2 |

As can be seen from Table 2, compared with Comparative Embodiment 1 which only uses the core assembly, Embodiment 1, Embodiment 3, and Embodiment 6 of the present disclosure which use a combination of the fixing device and the core assembly have better wearing comfort.

### Test Embodiment 3

Embodiment 1, Embodiment 3, Embodiment 6 and Comparative Embodiment 1 are tested for the degree of influence on facial appearance, and the scores are shown in the following Table 3: wherein, 1 score has the greatest impact on facial appearance and is unacceptable; 2 score has relatively great impact on facial appearance and is less unacceptable; 3 score has an impact on facial appearance, but is acceptable; 4 score has little impact on facial appearance; 5 score almost has no difference on facial appearance before and after wearing.

**Table 3**

| Subject | Embodiment 1 | Embodiment 3 | Embodiment 6 | Comparative Embodiment 1 |
|---|---|---|---|---|
| 1 | 4 | 5 | 5 | 5 |
| 2 | 5 | 5 | 5 | 5 |
| 3 | 4 | 4 | 4 | 4 |
| 4 | 4 | 4 | 4 | 4 |
| 5 | 4 | 4 | 4 | 4 |

As can be seen from the above table, compared with Comparative Embodiment 1 which only uses the core assembly, Embodiment 1, Embodiment 3, and Embodiment 6 of the present disclosure which use a combination of the fixing device and the core assembly have similar impact on facial appearance when wearing an oral cavity medication delivery device, that is, the oral cavity medication delivery device of the present embodiment has a similar wearing aesthetics compared to the comparative embodiment.

Although specific embodiments of the present disclosure have been described above, those skilled in the art will appreciate that these are merely illustrative and that various changes or modifications may be made to these embodiments without departing from the principles and essence of the present disclosure. Therefore, the scope of protection of the present disclosure is defined by the appended claims.

## Claims

1. An oral cavity medication delivery device, **characterized in that** it comprises a core assembly and a fixing device, and the core assembly comprises a molar matching functional area and a drug holding functional area which are integrally formed;
when the oral cavity medication delivery device is fixed in the oral cavity, the drug holding functional area is located in the space between the teeth and the cheek, or the drug holding functional area is located in the space between the teeth and the tongue;
the molar matching functional area comprises a first section close to the drug holding functional area and a second section far away from the drug holding functional area; one end of the first section and the second section are connected to each other, and the other end is an open end, they forms a U-shaped structure; the first section and the second section are used to anastomose with the buccal and lingual sides of the teeth respectively, and are used to fix the drug holding functional area on the teeth;
the drug holding functional area comprises a first ring and a second ring arranged coaxially; the axial clamping space formed between the first ring and the second ring is used to fix the drug;
the fixing device is a fixator or a fixing layer, which is used to strengthen the fixing effect of the core assembly on the teeth;
when the fixing device is the fixator:
when the oral cavity medication delivery device is in use, the lingual, buccal and occlusal surfaces of the fixator correspondingly match the lingual, buccal and occlusal surfaces of the teeth and cover the outer peripheral surfaces of the teeth; the fixator simultaneously covers the molar matching functional area;
when the fixing device is the fixing layer:
the fixing layer is attached to the outer surfaces of the first section and the second section for contact with teeth.

2. The oral cavity medication delivery device as defined in claim 1, wherein, the fixator and the molar matching functional area are connected by means of mechanical assembly, mechanical connection or adhesive bonding;
and/or, the fixing layer is further attached to other outer surfaces of the first section and the second section;
and/or, the first ring is close to the open end of the molar matching functional area, and the second ring is far away from the open end of the molar matching functional area, the first ring and the second ring are solid or hollow ring structures.

3. The oral cavity medication delivery device as defined in claim 2, wherein, the mechanical assembly is embedment, and slotted holes are provided in the fixator at a location corresponding to the drug holding functional area to achieve removable fixation between the fixator and the core assembly;
and/or, the mechanical connection is welding, riveting or bolting;
and/or, the adhesive is one or more selected from pressure-sensitive adhesive, starch glue, latex, epoxy resin and polyurethane;
and/or, the hollow ring structure is a closed ring or an open ring;
and/or, the shape of the ring structure is one or more selected from circle, ellipse, and polygon;
and/or, the shape of the ring structures of the first ring and the second ring are circular;
the hollow area of the first ring is smaller than that of the second ring.

4. The oral cavity medication delivery device as defined in at least one of claims 1-3, wherein, the material of the core assembly comprises one or more selected from titanium, stainless steel, cobalt-chromium alloy, cobalt-chromium-molybdenum alloy or precious metal, preferably cobalt-chromium alloy;
and/or, the material of the fixator is one or more selected from polyvinyl chloride, polyethylene terephthalate, polyethylene terephthalate-1,4-cyclohexanedimethpolyvinyl chloride, polyurethane, polyamide, ethylene-vinyl acetate copolymer, polycaprolactone, polyethylene, polypropylene, epoxy acrylate, methacrylate, and polyurethane acrylate; preferably polyethylene terephthalate or ethylene-vinyl acetate copolymer;
and/or, the length of the fixator corresponds to the length of 4-16 teeth in the maxillary or mandible, and preferably corresponds to the length of 5-9 teeth in the maxillary or mandible;
and/or, the length of the molar matching functional area corresponds to the length of 2-5 teeth in the mandible.

5. The oral cavity medication delivery device as defined in at least one of claims 1-4, wherein, the material of the fixing layer is one or more selected from polyvinyl chloride, polyethylene terephthalate, polyethylene terephthalate-1,4-cyclohexanedimethpolyvinyl chloride, polyurethane, polyamide, ethylene-vinyl acetate copolymer, polycarbonate, high-density polyethylene, polypropylene, cellulose acetate, hydroxypropyl cellulose and copovidone; preferably, polyethylene terephthalate or copovidone;
and/or, the thickness of the fixing layer is 0.01mm-1mm.

6. The oral cavity medication delivery device as defined in at least one of claims 1-5, wherein, the fixator is connected to the molar matching functional area in an embedded manner; the length of the molar matching functional area corresponds to the length of the mandibular first and second premolars and the first and second molars; the first ring and the second ring are circular closed rings;
and/or, the fixator is connected to the molar matching functional area in an embedded manner; the length of the molar matching functional area corresponds to the length of the mandibular first and second premolars and the first and second molars; the first ring and the second ring are elliptical closed rings;
and/or, the fixator is connected to the molar matching functional area in an embedded manner; the length of the molar matching functional area corresponds to the length of the maxillary second premolar and the first, second, and third molars; the first ring and the second ring are circular closed rings;
and/or, the fixator is connected to the molar matching functional area by pressure-sensitive adhesive as an adhesive; the length of the molar matching functional area corresponds to the length of the first, second, and third molars; the first ring and the second ring are polygonal closed rings;
and/or, the fixing layer is attached to entire outer surfaces of the first section of the U-shaped structure and the second section of the U-shaped structure; the length of the molar matching functional area corresponds to the length of the mandibular first and second premolars and the first and second molars; the first ring and the second ring are circular closed rings;
and/or, a pair of clamping walls are symmetrically provided on the first ring in the direction of the open end of the molar matching functional area.

7. A manufacturing method for the oral cavity medication delivery device as defined in at least one of claims 1-6, **characterized in that** it comprises the following steps: when the fixing device is a fixator, combining the fixator with the integrally formed first section and the second section in a wrapped form; when the fixing device is a fixing layer, the fixing layer is attached to the outer surfaces of the integrally formed first section and second section.

8. The manufacturing method for the oral cavity medication delivery device as defined in claim 7, wherein, a method for preparing the core assembly comprises laser casting, injection molding or impression molding;
and/or, a method for preparing the fixator comprises thermoforming, impression, cutting or 3D printing;
and/or, a method for preparing the fixing layer comprises dipping, spraying or thermoforming;
and/or, the wrap structure of the fixator and the first section and the second section is preferably formed by mechanical assembly, mechanical connection or adhesive bonding.

9. The manufacturing method for the oral cavity medication delivery device as defined in claim 7 and/or claim 8, wherein, in the method for preparing the fixator:
the steps of thermoforming are as follows: heating and softening the raw material of the fixator through a thermoforming machine and then vacuuming, and covering the raw material of the fixator on the dental mold and the molar matching functional area;
or, the steps of impression are as follows: heating and softening the raw material of the fixator, and covering it on the subject's teeth and the molar matching functional area;
and/or, in the method for preparing the fixing layer:
the steps of dipping are as follows: after dissolving the raw material of the fixing layer, immersing the molar matching functional area into the raw material solution of the fixing layer, taking it out, and drying it to form a membrane;
or, the steps of spraying are as follows: after dissolving the raw material of the fixing layer, coating it to the molar matching functional area by spraying, and drying it to form a membrane;
or, the steps of thermoforming are as follows: heating and softening the raw material of the fixing layer, covering it on the anastomosis functional area, and then cooling it;
and/or, the mechanical assembly is in the form of embedment;
and/or, the mechanical connection is in the form of welding, riveting or bolting;
and/or, the coating position of the adhesive is the outer surfaces of the first section and the second section for contact with the teeth, and preferably is the entire outer surfaces of the first section and the second section;
and/or, the coating thickness of the adhesive is no more than 1.0 mm.

10. A use of the oral cavity medication delivery device as defined in at least one of claims 1-6, **characterized in that**:
when the fixing device is a fixator, anastomosing the lingual, buccal and occlusal sides of the fixator with the lingual, buccal and occlusal surfaces of the teeth and wrapping around the peripheral surfaces of the teeth; placing the medication carrying functional area in the space between the teeth and the cheek, or placing the medication carrying functional area in the space between the teeth and the tongue; and wrapping the molar matching functional area with the fixator;
when the fixing device is a fixing layer: placing the medication carrying functional area in the space between the teeth and the cheek, or placing the medication carrying functional area in the space between the teeth and the tongue.
